# EUROPEAN PATENT APPLICATION

(11) **EP 4 691 422 A1**
(43) Date of publication of application: **11.02.2026**
(21) Application number: 24932679.4
(22) Date of filing: 30.09.2024
(51) Int. Cl.: A61F 2/08, A61B 17/80, A61B 17/56

(54) **ADJUSTABLE FULLY-FLEXIBLE LOOP PLATE FIXATION APPARATUS**

(30) Priority: 27.03.2024 CN 202410355015
(71) Applicant: STAR SPORTS MEDICINE CO., LTD., Beijing 100176 (CN)
(72) Inventor: DONG, Wenxing, Beijing 100176 (CN); GUO, Tingting, Beijing 100176 (CN); YIN, Jichen, Beijing 100176 (CN); LI, Zhengrong, Beijing 100176 (CN)
(74) Representative: Angerhausen, Christoph
(86) International application number: PCT/CN2024/123020
(87) International publication number: WO 2025/200369

(57) **Abstract**

The present application relates to an adjustable and fully flexible loop-band fixation device including a fixation band, a fixation loop, and a traction thread which are all made of flexible woven textile material. A wound tightening structure formed by interlacing of a pull suture in the middle and two sides of the fixation band fits an 8-shaped adjustable coil formed by extending on a side of the fixation band; and the 8-shaped adjustable coil has an anti-reverse structure and a self-interlacing structure, which form the fixation loop as a whole.

## Description

### CROSS-REFERENCE TO RELATED APPLICATION

The present application claims priority to Chinese Patent Application No. 202410355015.3, filed on March, 27, 2024, entitled "ADJUSTABLE AND FULLY FLEXIBLE LOOP-BAND FIXATION DEVICE", which is incorporated herein by reference in its entirety.

### TECHNICAL FIELD

The present application relates to the technical field of medical devices, and particularly, to an adjustable and fully flexible loop-band fixation device.

### BACKGROUND

The existing suspension fixation device for ligament repair mainly includes the titanium plate fixation band and the suture coil which belong to the combination of metal material and suture material. Due to the stiffness difference of the two materials, the overall stability of the suspension fixation is insufficient; for example, the wear of the suture coil caused by the metal material may lead to fixation failure, and the hard-on-hard contact between the metal material and the bone may lead to windshield wiper effect, bungee jumping effect, and the like. In addition, due to the poor deformation capacity of the metal material, before surgery, it is necessary to drill a bone tunnel enough for the metal material to pass through, and this causes relatively great damage to the bone; after surgery, the metal causes foreign body sensation on the bone surface and has poor biocompatibility. Further, the coil fitting the fixation device is mainly a non-adjustable length band or an adjustable length band that need additional fixation. During the surgery, a suitable specification of the coil fitting the fixation device needs to be selected by measurement and calculation, or other operations such as knotting and implanting screws need to be used to assist in fixation, reducing the surgery efficiency.

There have been techniques in which the suture material is used in the fixation band. For example, CN116869701A discloses a deformable body and a fully flexible fixation device including the deformable body, the flexible anchor of the fixation device consists of the hardened segment and the non-hardened segment which are alternately arranged, and the suture of the hardened segment is hardened by heat melting. Although the suture fixation device is used in this technology, the joint between the hardened segment and the non-hardened segment of the suture fixation device still leads to the risk of wear and failure due to the poor stiffness. For example, CN114869374A discloses a loop in ligament or tendon injury repair surgery. The length of the loop is not adjustable, and folding and contraction of the thread band structure after stress is uncontrollable, so that the thread band is easily pulled into the bone tunnel due to unbalanced stress, leading to the fixation failure and surgery failure.

### SUMMARY

One of the objects of the present application is to provide an adjustable and fully flexible loop-band fixation device that has desired structural stability to solve the problem that it is difficult for the existing suspension and fixation band plate to achieve the stable structure through the fully flexible state, and the length of the coil is not adjustable or the adjustable coil cannot provide firm support alone.

In order to achieve the above object, the present application provides an adjustable and fully flexible loop-band fixation device including a fixation band, a fixation loop, and a traction thread which are all made of flexible woven textile material, where the fixation loop is formed by repeated interlacing of a pull suture through the fixation band; an interlacing structure of the pull suture on an upper surface of the fixation band along a thickness direction forms a tightening loop of the fixation loop; an adjustable coil of the fixation loop is formed on a lower surface of the fixation band along the thickness direction by interlacing and extending, after the tightening loop is formed, two free ends of the pull suture intersect with each other along opposite directions and interlace through themselves to form the adjustable coil, and the adjustable coil includes an 8-shaped structure formed by intersecting and interlacing twice; the pull suture is provided with a self-locking knot on the upper surface of the fixation band along the thickness direction, and after the adjustable coil is formed, the two free ends reversely pass through the fixation band and wind back through the tightening loop to form the self-locking knot; and the traction thread is threaded through the fixation band and is used to draw the fixation band against a surface of a cortical bone.

According to the embodiments in the first aspect of the present application, the fixation band includes at least two layers of woven flat straps, and the woven flat straps overlap with each other and have at least a certain length.

According to any one of the embodiments in the first aspect of the present application, the tightening loop includes a plurality of loops provided on the fixation band, the plurality of loops are crisscrossed longitudinally and laterally on a stress-bearing wall of the fixation band and formed by repeated interlacing of the pull suture through the fixation band; the tightening loop includes a median segment and a side segment; and the median segment is an initial interlacing segment of the pull suture through the fixation band, and the median segment is provided in a right middle of the fixation band along a length direction and extends along a width direction of the fixation band.

According to any one of the embodiments in the first aspect of the present application, two free ends of the median segment pass through an entire thickness of the fixation band, after first intersection on the other side of the fixation band, and the two free ends of the median segment reversely pass through a single-layer woven flat strap of the fixation band, and then pass through the fixation band entirely to form the side segment.

According to any one of the embodiments in the first aspect of the present application, the adjustable coil includes an anti-reverse structure located at the bottom, and the two free ends of the pull suture interlace through their own segment after first intersection to obtain the anti-reverse structure with two interlocked loops.

According to any one of the embodiments in the first aspect of the present application, the adjustable coil includes an intersection point located above the anti-reverse structure, and the two free ends of the pull suture extend after their own suture passes through a certain length and form the intersection point after second intersection; and the two free ends interlace through each other's initial segment after intersection, and pass out after passing through a certain length to form a complete adjustable coil.

According to any one of the embodiments in the first aspect of the present application, after the adjustable coil is formed, the two free ends of the pull suture pass through the entire thickness of the fixation band and wind back through the side segment to form the self-locking knot.

According to any one of the embodiments in the first aspect of the present application, one of the free ends of the traction thread winds back through the width direction of the fixation band to form a U-shaped structure of the traction thread, and the traction thread is connected to the fixation band in a same plane.

According to any one of the embodiments in the first aspect of the present application, the fixation band is formed by folding the woven flat strap in half along its width direction and has a U-shaped longitudinal section; or the fixation band is formed by closing the woven flat straps along the width direction of the fixation band and has an O-shaped longitudinal section.

According to any one of the embodiments in the first aspect of the present application, the adjustable and fully flexible loop-band fixation device further includes a loop-band structure connected to the bottom of the adjustable coil, and the anti-reverse structure is located at a bottom of the loop-band structure.

The fixation band, the fixation loop, and the traction thread which are all made of flexible woven textile material form the fully flexible suture structure, which may reduce the wear and stress concentration caused by the stiffness difference of the parts, and may reduce the wear of the bone tunnel and surrounding tissue caused by the fixation component, reducing the pain of the patient. The fully flexible suture structure may deform when passing through the bone tunnel to reduce the amount of bone removed, thereby reducing iatrogenic injury and facilitating recovery after the surgery.

The fixation loop formed by repeated interlacing through the fixation band can form different coils or structures, which is beneficial for tightening and deformation of the fixation band and fixation and connection of the graft.

The pull suture repeatedly interlaces through the outer surface of the fixation band to form the tightening loop of the fixation loop, ensuring the stability of the deformation of the fixation band in the three-dimensional space.

After the tightening loop is formed, the free ends of the pull suture extend along the other side of the fixation band, intersect twice, and interlace through themselves to form the adjustable coil with the 8-shaped structure, so that the length of the coil can be adjusted, and the anti-reverse effect of the coil can be increased; the intersection point in the middle of the 8-shaped adjustable coil can make the balanced force applied to the pull sutures on two sides, so that the fixation band always keeps perpendicular to the axial tensile force and is the fully fitted to the surface of the cortical bone to achieve stable fixation.

After the adjustable coil is formed, two free ends of the pull suture reversely pass through the fixation band and wind back through the tightening loop to form the self-locking knot, the self-locking knot is locked to the upper surface of the fixation band by the tightening loops on two sides of the fixation band, and the degree of the coil contraction may be adjusted by tightening the free ends of the self-locking knot to achieve the self-locking function. The structure of the self-locking knot omits the step of knot tying in the surgery and reduces the surgery duration, and its firmness and anti-slip-off capacity may provide stable and continuous fixation for surgical repair, ensuring the effect of surgical repair.

The traction thread can draw the fixation band against the surface of the cortical bone through the bone tunnel, reducing the risk of slipping off of the fixation band and increasing the success rate of the surgery.

### BRIEF DESCRIPTION OF THE DRAWINGS

In order to illustrate technical solutions of embodiments of the present application more clearly, drawings used to describe the embodiments of the present application are introduced briefly below. It should be understood that the drawings described below only show some embodiments of the present application, and thus should not be regarded as a limitation of the scope. For those skilled in the art, other related drawings may be obtained from these drawings without inventive efforts.
Fig. 1 is a formal schematic structural view of an entire adjustable and fully flexible loop-band fixation device according to the present application;
Fig. 2 is a schematic perspective view of an entire adjustable and fully flexible loop-band fixation device according to the present application;
Fig. 3 is an overall schematic structural view of an fixation loop according to the present application;
Fig. 4 is a schematic structural view of a connection between a pull suture and a fixation band;
Fig. 5 is a schematic view of a forming process of an anti-reverse coil on an adjustable coil;
Fig. 6 is a schematic view of a forming process of an intersection part on an adjustable coil;
Fig. 7 is a schematic view of a forming process of a coil on an adjustable coil;
Fig. 8 is a schematic view of a forming process of a self-locking knot;
Fig. 9 is a schematic view of steps of a surgical operation in Embodiment 1;
Fig. 10 is a schematic structural view of a fully flexible loop-band fixation device in Embodiment 2; and
Fig. 11 is a schematic structural view of a fully flexible loop-band fixation device in Embodiment 3.

Reference signs:
100: Fixation band; 110: Fixation band plate;
200: Fixation loop;
210: Tightening loop; 211: Median segment; 212: Side segment;
220: Adjustable coil; 221: Anti-reverse coil; 222: Self-locking knot; and
300: Traction thread.

### DETAILED DESCRIPTION

In order to make the purpose, technical solutions, and advantages of embodiments of the present application more clear, the technical solutions in the embodiments of the present application will be described clearly and completely below with reference to the drawings in the embodiments of the present application Obviously, the described embodiments are only some but not all of the embodiments of the present application. The components in the embodiments of the present application generally described and shown in the drawings herein may be arranged and designed in various different configurations.

It should be noted that, in the description of the embodiments of the present application, the orientation or positional relationship indicated by the terms such as "outer" and "inner" is based on the orientation or positional relationship shown in the drawings, or may be the orientation or positional relationship that the product of the present application is commonly placed in use, and is only for the convenience of describing the present application and simplifying the description, and does not indicate or imply that the described device or element must have a specific orientation or must be constructed and operated in a specific orientation, and thus cannot be understood as a limitation of the present application. In addition, the terms such as "first" and "second" are only used for distinguishing descriptions, and cannot be understood as indicating or implying relative importance.

In the description of the present application, it should be noted that, unless otherwise expressly specified and limited, the terms such as "provided" and "connected" should be understood in a broad sense. For example, they may refer to a fixed connection, a detachable connection or an integral connection, or may be a direct connection, an indirect connection by an intermediate medium, or an internal communication between two elements. For those of ordinary skill in the art, the specific meanings of the above terms in the present application may be understood in accordance with specific conditions.

An adjustable and fully flexible loop-band fixation device according to the present application is mainly configured to suspend and fix the ligament graft, change the shape of a component of the fixation device using features of a suture such as flexibility, and stabilize deformation of a fixation band plate by providing a tightening loop and an adjustable coil, so that adjustment to a fixation loop is controllable, and fixation strength of the fixation component is increased as a whole.

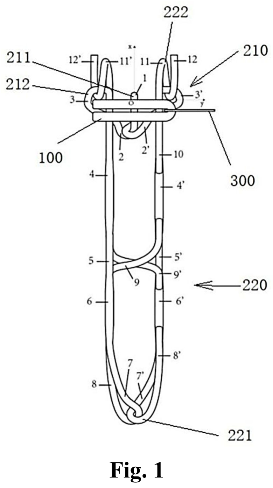

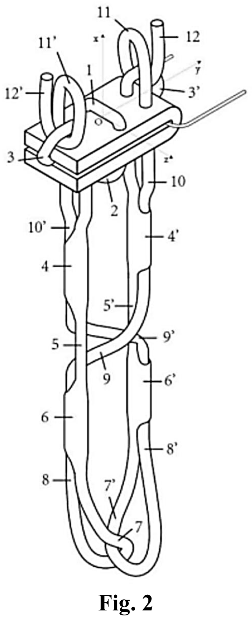

Referring to Fig. 1, Fig. 2, and Fig. 3, the adjustable and fully flexible loop-band fixation device according to the present application includes the fixation band 100, the fixation loop 200, and the traction thread 300 which are all made of the flexible woven textile material, and the fixation loop 200 is specifically formed by repeated interlacing of the suture through the fixation band 100. The parts made of the flexible woven textile material form the fully flexible suspension and fixation device.

The fully flexible suture structure may reduce the wear and stress concentration caused by the stiffness difference of the fixation band 100, the fixation loop 200, and the traction thread 300, and may reduce the wear of the bone tunnel and surrounding tissue caused by the fixation component, reducing the pain of the patient.

The fully flexible suture structure has desired deformability and may deform when passing through the bone tunnel to reduce the amount of bone removed, thereby reducing iatrogenic injury and facilitating recovery after the surgery.

The repeated interlacing structure of the pull suture on the upper surface of the fixation band 100 along the thickness direction forms the tightening loop 210 of the fixation loop 200. Referring to the structure in the drawing, the tightening loop 210 is provided on the upper outer surface and the lower outer surface of the fixation band 100 and includes the wound distribution structure along the lateral direction and longitudinal direction. The tightening loop 210 can control the fixation band 100 to stably compress and deform under the tightening force of the pull suture, so that the pulling points on the fixation band 100 have even stress distribution, ensuring that the stability of the deformation of the fixation band 100 under the solid angle.

The other side of the fixation band 100 relative to the tightening loop 210 is provided with the adjustable coil 220. Referring to the drawing, after the tightening loop 210 is formed, two free ends of the pull suture intersect with each other along opposite directions after passing through the overall thickness direction of the fixation band 100 and interlace through themselves to form the adjustable coil 220. In the drawing, the adjustable coil 220 is disposed under the fixation band 100, and the adjustable coil 220 includes the 8-shaped structure formed by intersecting and interlacing twice.

The main purpose of the adjustable coil 220 with the 8-shaped structure is to freely adjust the length of the coil for fixing the graft, so that the operation of the surgical process is controllable, increasing the surgery efficiency.

The upper surface of the fixation band 100 along the thickness direction is provided with the self-locking knot 222. After the adjustable coil 220 is formed under the fixation band 100, two free ends of the fixation loop 200 reversely pass upward through the fixation band 100 and wind back through the tightening loop 210 to form the self-locking knot 222. The self-locking knot 222 is provided on the upper surface of the fixation band 100. Specifically, after the self-locking knot 222 with the 6-shaped structure adjusts the length of the 6-shaped coil and tightens the fixation band 100, the self-locking loop is locked to the upper surface of the fixation band 100 by the tightening loops 210 on two sides of the fixation band 100, and the degree of coil contraction may be adjusted by tightening the free ends of the 6-shaped coil to achieve the self-locking function.

In the present application, since the knot is formed by double force squeezing, and is firm and not easily slips off, the self-locking knot 222 omits the step of knot tying in the surgery and reduces the surgery duration, and its firmness and anti-slip-off capacity may provide stable and continuous fixation for surgical repair, ensuring the effect of surgical repair.

The traction thread 300 is threaded through the fixation band 100 and is used to draw the fixation band 100 against the surface of the cortical bone after passing through the bone tunnel, reducing the risk of slipping off of the fixation band 100 and increasing the success rate of the surgery.

In the present application, the fixation band 100 includes at least two layers of woven flat straps, and the woven flat straps overlap with each other and have at least a certain length. Specifically, the woven flat straps can form a long strip structure with a certain length after being fitted, which is beneficial for winding of the tightening loop 210 around the fixation band 100 and forming of the adjustable coil 220 under the fixation band 100 by interlacing.

In one of the arrangements, the fixation band 100 is formed by folding the woven flat strap with the width of 4 mm in half along its width direction, so that the fixation band 100 has the U-shaped longitudinal section as a whole and has one open end and one closed end.

The structure for drawing the fixation band 100 is formed by the traction thread 300 threading through the fixation band 100, and the traction thread 300 is a high-performance nonabsorbable 2 # suture with the diameter ranging from 0.500 mm to 0.599 mm. Specifically, one of the free ends of the traction thread 300 winds back through the closed end of the U-shaped fixation band 100 and passes through the closed end along the width direction of the fixation band 100 to form another U-shaped structure formed by the traction thread 300. The closed ends of two U-shaped structures are connected to each other in the same plane, so that the traction thread 300 is connected to the fixation band 100 in the same plane.

The traction thread 300 is in contact with the fixation band 100 along the entire width direction, so that under a condition that the traction thread 300 draws through the bone tunnel, the fixation band 100 has more even stress distribution, reducing the risk of slipping off and increasing the success rate of the surgery.

The tightening loop 210 includes a plurality of loops provided on the fixation band 100. Specifically, the plurality of loops are crisscrossed longitudinally and laterally on the stress-bearing wall of the fixation band 100 and formed by repeated interlacing of the pull suture through the fixation band 100.

Further, the tightening loop 210 includes the median segment 211 and the side segment 212. During interlacing process, two free ends of the pull suture interlace along the width direction of the fixation band 100 and are located at the center along the length direction. The median segment 211 parallel to the width of the fixation band 100 is formed on one of the side surfaces of the fixation band 100, and the median segment 211 is an initial interlacing segment of the pull suture through the fixation band 100.

In forming of the median segment 211 by interlacing, two free ends of the pull suture pass through the entire thickness of the fixation band 100, that is, after passing through two layers of woven flat straps, two free ends of the pull suture intersect with each other on the other side surface of the fixation band 100, then reversely pass through the single-layer woven flat strap of the fixation band 100 to form a longitudinal loop in the middle of the other side of the fixation band 100; then two free ends of the pull suture pass through the entire two layers of woven flat straps of the fixation band 100 to form the lateral side segments 212 located on two sides of the fixation band 100 along the length direction.

The median segment 211 and the side segment 212 form the tightening loop 210 formed by interlacing of the pull suture through the upper surface of the fixation band 100, and the tightening loop 210 is crisscrossed laterally and longitudinally in three points, so that the pulling points on the fixation band 100 have even stress distribution, and the crossed longitudinal loop structure of the pull suture at the bottom of the fixation band 100 uniformly transmits the force applied to the middle stressed point of the fixation band 100 to two sides. Therefore, the pull suture is tightened winding the upper surface and the bottom of the fixation band 100, ensuring the stability of lateral deformation of the fixation band 100.

The lateral loops of the pull suture on two sides of the fixation band 100 pass upward into the fixation band 100 from the bottom of the fixation band 100, pass out from the side surface after passing through the single-layer woven flat strap of the fixation band 100, and pass downward through the fixation band 100 from the top of the fixation band 100 to pass out from the bottom; passing through the single-layer fixation band 100 may restrain the side segment 212, that is, the relative position of the lateral loop on the fixation band 100 and the fixation band 100, ensuring that the loop will not slip to the long side of the fixation band 100 and ensuring the stability of longitudinal deformation of the fixation band 100.

Therefore, under a condition the pull suture tightens, the fixation band 100 may deform stably in the whole space under the action of the pull suture, and the formed solid structure is fixed to the opening of the bone tunnel, which is fixed and stable and increases the consistency and controllability of the knotting effect.

The entire adjustable coil 220 is provided under the fixation band 100 and includes the anti-reverse structure located at the bottom. After the tightening loop 210 is formed, two free ends of the pull suture passing through entire the fixation band 100 intersect for the first time, then interlace through their own segment after the first intersection to obtain the anti-reverse coil 221 with two interlocked loops, forming the specific form of the anti-reverse structure.

The adjustable coil 220 includes the intersection point located above the anti-reverse coil 221, and two free ends of the pull suture extend after their own suture passes through a certain length and intersect for the second time to form the intersection point located in the middle of the adjustable coil 220 after.

Two free ends interlace through each other's initial segment after the second intersection, and extend after passing through a certain length to form the complete adjustable coil 220.

In the forming process of the adjustable coil 220, two free ends of the pull suture intersect for the first time and interlace through their own line segment to form the anti-reverse coil 221, and pass out from their own segment after interlacing themselves for a certain length, next intersect for the second time to form the intersection point in the middle of the adjustable coil 220, then pass into the fixation loop 200 located on each other's the other segment along the direction of after the second intersection, and finally extend from each other's segment after passing through a certain length, completing the forming of the adjustable coil 220.

Under a condition that the reverse pulling force is applied to the adjustable coil 220 by the anti-reverse structures intersecting at the bottom of the 8-shaped adjustable coil 220, balanced force is applied to two sides of the loop at the bottom, and it is difficult for the bottom end to contract, increasing the anti-reverse effect of the adjustable coil 220.

Under a condition that the fixation loop 200 is pulled along the positive direction to contract the coil, the intersection point located in the middle of the 8-shaped adjustable coil 220 may limit the unilateral displacement of the suture on two sides. No matter the positive pulling force or the negative pulling force is applied to the coil, balanced force is applied to the fixation loops 200 on two sides, so that the fixation band 100 always keeps perpendicular to the axial tensile force and is fully fitted to the surface of the cortical bone to achieve stable fixation.

After intersecting for the first time, two free ends of the pull suture interlace through their own segment for a certain length; after intersecting for the second time, two free ends of the pull suture interlace through each other's segment for a certain length to form four self-interlacing structures of the adjustable coil 220. Under a condition that the positive tensile of the pull suture is applied to the coil, four self-interlacing structures contract to serve the function of adjusting the length of the coil.

After the adjustable coil 220 is formed, two free ends of the pull suture reversely interlace through the entire thickness of the fixation band 100 and wind back through the side segment 212 to form the self-locking knot 222.

Specifically, the self-locking knot 222 is formed on the plane above the fixation band 100 and has the 6-shaped structure; after the length of the 6-shaped self-locking coil is adjusted and the fixation band 100 is tightened, the self-locking loop is locked to the upper surface of the fixation band 100 by the loops on two sides of the fixation band 100, that is, by the side segments 212, and the degree of coil contraction may be adjusted by tightening the free ends of the 6-shaped self-locking loop to achieve the self-locking function.

Since the knot is formed by double force squeezing, and is firm and not easily slips off, the arrangement of the self-locking knot 222 omits the step of knot tying in the surgery and reduces the surgery duration, and its firmness and anti-slip-off capacity may provide stable and continuous fixation for surgical repair, ensuring the effect of surgical repair.

Based on the above structure and the forming process, the entire fixation loop 200 is divided into the upper part and the lower part by the plane where the fixation band 100 is located; the fixation loop 200 located under the fixation band 100 is the 8-shaped adjustable coil. The pulling structure under the fixation band 100 is formed by interlacing of the pull suture through the U-shaped fixation band 100 and interlacing of the pull suture through its own segment, and the fixation loop 200 located above the fixation band 100 is the tightening loop 210; in the forming process, the fixation loop 200 first forms the tightening loop 210, and then forms the adjustable coil 220.

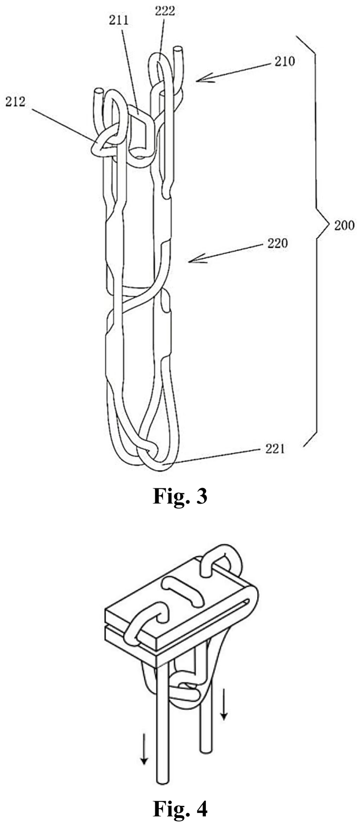

Specifically, firstly, the pull suture interlace along the width direction of the fixation band 100 and are located at the center along the length direction, the segment parallel to the width is formed on one surface of the U-shaped fixation band 100, and free ends of two sutures located on the other surface of the U-shaped fixation band 100 intersect and pass back through the U-shaped fixation band 100, so that a longitudinal loop is formed in the middle of the fixation band 100, and two lateral loops are formed on two sides of the fixation band 100, for which reference may be made to Fig. 4.

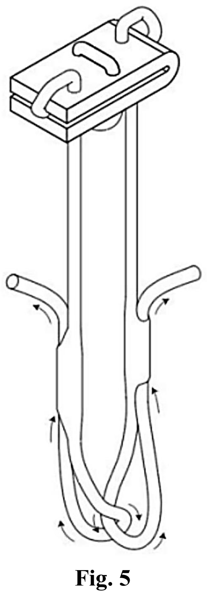

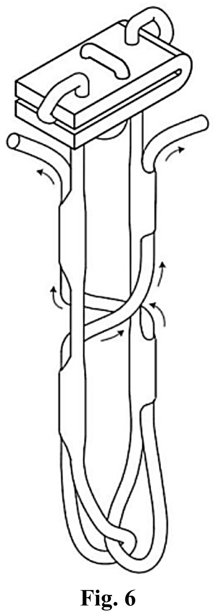

Secondly, referring to Fig. 5 to Fig. 7, the lower coil in the 8-shaped adjustable coil is formed by the self-interlacing pull suture passing out below the plane where the fixation band 100 is located, and the jointed intersection part in the middle is formed by the pull sutures intersecting on two sides along the diagonal direction and then interlacing through each other's the initial end; the upper coil is formed by the pull suture passing upward and back through the fixation band 100.

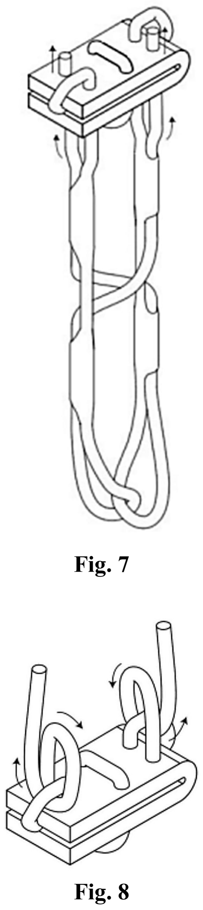

Finally, the free ends of the pull suture pass out from the side edges of two lateral loops formed by the pull suture and the fixation band 100, respectively, and pass through the lateral loops from top to bottom to form the 6-shaped self-locking knot 222, and the forming of the self-locking knot 222 is shown in Fig. 8.

Different embodiments will be used for further illustrating different components of the adjustable and fully flexible loop-band fixation device according to the present application.

### Embodiment 1

Referring to Fig. 1 to Fig. 3, the adjustable and fully flexible loop-band fixation device in this embodiment consists of the fixation band 100, the fixation loop 200, and the traction thread 300, the fixation band 100 and the traction thread 300 are thread in double U shape, and the fixation loop 200 and the fixation band 100 are connected by interlacing and winding. The midpoint of the pull suture is the midpoint of the segment 1, starts from the center point O of the upper surface of the fixation band 100, and passes through the fixation band 100 or the pull suture itself in sequence based on the order from 1 to n, where n is a positive integer greater than 1, the segment n and the segment n' are in circular symmetry with respect to the longitudinal axis perpendicular to the upper surface of the fixation band 100 where the point O is located; under a condition that the pull suture interlaces around and is connected to the fixation band 100, the contact positions between the traction thread 300 and the fixation band 100 are always fitted to each other, and the pull suture does not pass through the traction thread 300 or the contact gap between the traction thread 300 and the fixation band 100.

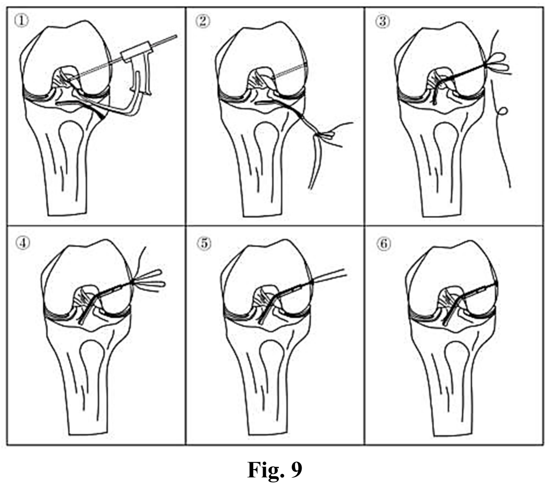

The steps of the surgical operation in this embodiment are shown in Fig. 9, and the repair of the anterior cruciate ligament of the knee joint is given as an example; the fixation band 100 passes through the bone tunnel and reaches the surface of the cortical bone with the stretching of the traction thread 300, the length of the coil is adjusted by stretching the loop on the self-locking knot 222, the graft is drawn to the appropriate position in the bone tunnel, and the tightening coil compresses the fixation band 100 into the knot, then the thread end on the self-locking knot 222 is stretched to tighten the self-locking knot 222 to get a stable structure which is knot-free and does not have loosened knot, finally, the excess pull suture is cut off, completing the suspension and fixation for ligament repair.

### Embodiment 2

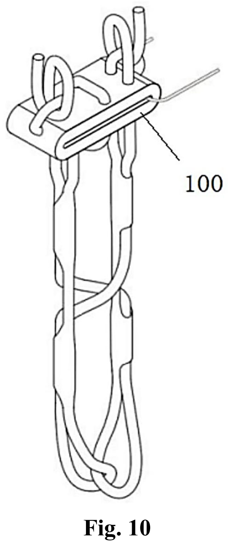

As shown in Fig. 10, the component for the fully flexible knot-free suspension and fixation band 100 is basically the same as that in the Embodiment 1, and the difference is that the fixation band 100 is formed by closing the woven flat straps along the width direction of the fixation band and has an O-shaped longitudinal section.

### Embodiment 3

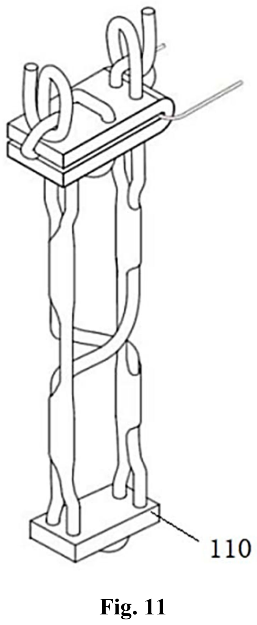

As shown in Fig. 11, the adjustable and fully flexible loop-band fixation device in this embodiment is basically the same as that in the Embodiment 1, and the difference is that the fixation band plate 110 is provided at the bottom of the adjustable coil 220 formed by the pull suture, and two intersected loops of the pull suture are located on the bottom surface of the fixation band plate 110.

In the steps of the surgical operation in this embodiment are, the tibiofibular symphysis ligament repair is given as an example; the fixation band 100 passes through the fibula and the tibial tunnel and reaches the surface of the cortical bone of the tibia with the stretching of the traction thread 300, the fixation band plate 110 is left on the surface of the cortical bone on the fibula side, and the length of the coil is adjusted by stretching the loop on the self-locking knot 222, so that the tibiofibular symphysis is completely reset, the fixation band 100 is fitted to the surface of the cortical bone of the tibia and contracts into the knot, then the thread end on the self-locking knot 222 is stretched to completely lock the self-locking knot 222, and the excess pull suture is cut off to complete the tibiofibular symphysis ligament repair.

It should be emphasized that in this solution, in conjunction with the operation mode of suspension and fixation in ligament repair surgery, the structural design of the full suture band-loop fixation device mainly achieves the following technical effects.
1. Full flexibility: compared with the loop plate made of metal fixture or the fixation band subjected to partial suture hardening treatment, the components of this solution are made of flexible sutures, so that can reduce the risk of wear between the components and between the bone tunnel and surrounding tissue may be reduced, the problem of uneven stress distribution caused by the stiffness difference may be reduced, and the stable and lasting fixation may be provided; also, the fully flexible fixation band plate may pass through the bone tunnel with the smaller diameter by deforming, so that the diameter required for the bone tunnel is reduced, and the iatrogenic injury is reduced.
2. Deformation consistency: the pull suture and the fixation band are connected to each other, so that the pull suture contracts, and the deformation of the fixation band is stable and consistent, solving the problem of failure of the flexible fixation band caused by the uncontrollable deformation and the susceptibility to the unbalanced structural deformation when force is applied.
3. Self-locking of the adjustable coil: the process of measuring, calculating, and selecting the band plate with appropriate specification during surgery is omitted, the process of additional knotting or auxiliary fixation of the adjustable coil by screw implantation is omitted; the knot may be formed by tightening the self-locking loop and the self-locking thread end in turn based on the steps, and the self-locking knot is fixed by the reverse force of the doublelayer coil with being firm and non-sliding, so that the stable and effective support may be provided, the accuracy and flexibility of the surgery may be increased, and the surgery duration may be greatly reduced. With the structural design of the full suture band plate, the steps of the surgical operation are reduced, the surgery efficiency is increased, and the risk of complications caused by the surgery is reduced; its feature of full flexibility may reduce the wear of the suture and surrounding tissue caused by the fixture, balance the overall stress, and reduce the risk of failure caused by stress concentration; its feature of deformation may reduce the diameter of the bone tunnel required during implantation, reduce iatrogenic injury, reduce the volume of the fixation band after implantation, and reduce the foreign body sensationand the risk of complications.

It should be noted that, the features in the embodiments of the present application may be combined with each other without conflict.

The above descriptions are only the preferred embodiments of the present application, and are not intended to limit the present application. For those skilled in the art, various modifications and variations may be made to the present application. Any modification, equivalent replacement and improvement made within the gist and principle of the present application shall be included in the protection scope of the present application.

## Claims

1. An adjustable and fully flexible loop-band fixation device, wherein the adjustable and fully flexible loop-band fixation device comprises a fixation band, a fixation loop, and a traction thread which are all made of flexible woven textile material, where the fixation loop is formed by repeated interlacing of a pull suture through the fixation band;
an interlacing structure of the pull suture on an upper surface of the fixation band along a thickness direction forms a tightening loop of the fixation loop; an adjustable coil of the fixation loop is formed on a lower surface of the fixation band along the thickness direction by interlacing and extending, after the tightening loop is formed, two free ends of the pull suture intersect with each other along opposite directions and interlace through themselves to form the adjustable coil, and the adjustable coil comprises an 8-shaped structure formed by intersecting and interlacing twice;
the two free ends of the pull suture interlace through their own segment after first intersection to obtain an anti-reverse structure with two interlocked loops;
the adjustable coil comprises an intersection point located above the anti-reverse structure, and the two free ends of the pull suture extend after their own suture passes through a certain length and form the intersection point after second intersection;
the two free ends interlace through each other's initial segment after the second intersection, and pass out after passing through a certain length to form a complete adjustable coil;
the pull suture is provided with a self-locking knot on the upper surface of the fixation band along the thickness direction, and after the adjustable coil is formed, the two free ends reversely pass through the fixation band and wind back through the tightening loop to form the self-locking knot; and
the traction thread is threaded through the fixation band and is configured to draw the fixation band against a surface of a cortical bone.

2. The adjustable and fully flexible loop-band fixation device according to claim 1, wherein the fixation band comprises at least two layers of woven flat straps, and the woven flat straps overlap with each other and have at least a certain length.

3. The adjustable and fully flexible loop-band fixation device according to claim 2, wherein the tightening loop comprises a plurality of loops provided on the fixation band, the plurality of loops are crisscrossed longitudinally and laterally on a stress-bearing wall of the fixation band and formed by repeated interlacing of the pull suture through the fixation band;
the tightening loop comprises a median segment and a side segment; and
the median segment is an initial interlacing segment of the pull suture through the fixation band, and the median segment is provided in a right middle of the fixation band along a length direction and extends along a width direction of the fixation band.

4. The adjustable and fully flexible loop-band fixation device according to claim 3, wherein two free ends of the median segment pass through an entire thickness of the fixation band, after first intersection on the other side of the fixation band, the two free ends of the median segment pass out from a side surface after reversely passing through a single-layer woven flat strap of the fixation band, pass downward through the fixation band entirely from a top of the fixation band to form the side segment.

5. The adjustable and fully flexible loop-band fixation device according to claim 1, wherein the adjustable coil comprises an anti-reverse structure located at a bottom.

6. The adjustable and fully flexible loop-band fixation device according to claim 3, wherein after the adjustable coil is formed, the two free ends of the pull suture pass through the entire thickness of the fixation band and wind back through the side segment to form the self-locking knot.

7. The adjustable and fully flexible loop-band fixation device according to claim 1, wherein one of the free ends of the traction thread winds back through the width direction of the fixation band to form a U-shaped structure of the traction thread, and the traction thread is connected to the fixation band in a same plane.

8. The adjustable and fully flexible loop-band fixation device according to claim 2, wherein the fixation band is formed by folding the woven flat strap in half along its width direction and has a U-shaped longitudinal section; or
the fixation band is formed by closing the woven flat straps along the width direction of the fixation band and has an O-shaped longitudinal section.

9. The adjustable and fully flexible loop-band fixation device according to claim 5, wherein the adjustable and fully flexible loop-band fixation device further comprises a loop-band structure connected to the bottom of the adjustable coil, and the anti-reverse structure is located at a bottom of the loop-band structure.
